Europäisches Patentamt

European Patent Office

Office européen des brevets

(11) Numéro de publication : **0 041 880**
**B1**

(12) **FASCICULE DE BREVET EUROPÉEN**

(45) Date de publication du fascicule du brevet :
11.05.83

(21) Numéro de dépôt : 81400815.7

(22) Date de dépôt : 22.05.81

(51) Int. Cl.³ : **A 61 K 39/12**, A 61 K 39/145,
**C 12 N 7/00**

(54) **Procédé pour isoler des antigènes glycoprotéiques viraux et son application à la préparation de vaccins.**

(30) Priorité : 05.06.80 FR 8012522

(43) Date de publication de la demande :
16.12.81 Bulletin 81/50

(45) Mention de la délivrance du brevet :
11.05.83 Bulletin 83/19

(84) Etats contractants désignés :
AT BE CH DE FR GB IT LI LU NL SE

(56) Documents cités :
EP A 0 001 365
EP A 0 001 838
FR A 11 168
FR A 2 297 635
CHEMICAL ABSTRACTS, vol. 84, no. 17, 26 avril
1976, résumé no. 118233r, page 250 COLUMBUS,
Ohio (US)
NATURE, vol. 276, no. 5689, décembre 1978
B. MOREIN et al. : « Effective subunit vaccines
against an enveloped animal virus » pages 715-
718

(73) Titulaire : **SYNTHELABO**
**58, rue de la Glacière**
**F-75621 Paris Cedex 13 (FR)**

(72) Inventeur : **Leclerc, Jean**
**35, Avenue Georges Pompidou**
**F-33500 Libourne (FR)**
Inventeur : **De Rudder, Jean**
**44, Avenue des Etats-Unis**
**F-78000 Versailles (FR)**

(74) Mandataire : **Polus, Camille et al**
**c/o Cabinet Lavoix 2, Place d'Estienne d'Orves**
**F-75441 Paris Cedex 09 (FR)**

Jouve, 18, rue St-Denis, 75001 Paris, France

### Procédé pour isoler des antigènes glycoprotéiques viraux et son application à la préparation de vaccins

La présente invention concerne la préparation d'antigènes extraits de virus, en particulier des virus de la grippe, dans le but de préparer des vaccins permettant d'immuniser l'homme ou les animaux contre ces virus.

Certains virus (Myxovirus, Paramyxovirus, Rhabdovirus, Virus des Leucoses, etc.) sont enveloppés d'une membrane lipidique dans laquelle sont fixées des glycoprotéines qui constituent, par exemple dans le cas du virus de la grippe, les antigènes permettant la vaccination contre ces virus.

Ces antigènes glycoprotéiques sont sélectivement solubilisés par diverses solutions aqueuses de détergents, en particulier des détergents non ioniques, par exemple le Triton®. On peut alors, par diverses méthodes telles que centrifugation, exclusion, etc. séparer ces antigènes solubilisés dans la solution de détergent, du reste de la particule virale dont les protéines ne présentent pas d'intérêt pour la préparation de vaccins.

Il faut ensuite séparer les glycoprotéines du détergent en conservant les propriétés immunologiques et fonctionnelles qui permettent de les caractériser : par exemple activité enzymatique de la neuraminidase et hémagglutinante de l'hémagglutinine. Ces glycoprotéines doivent être dosables par immunodiffusion radiale et se révéler immunogènes.

Pour séparer les glycoprotéines des détergents non ioniques, on a déjà proposé divers procédés. C'est ainsi que Scheid et coll. (Virology 50, 640-652, 1972) ont précipité les glycoprotéines en solution aqueuse par addition de butanol. Ceci entraîne une dénaturation partielle des glycoprotéines.

Holloway (Analytical Biochemistry 53, 304-308, 1973) a proposé d'utiliser des billes de copolymère styrène-divynylbenzène pour absorber le détergent non ionique, les glycoprotéines restant en solution dans la phase aqueuse. Cette technique est d'une mise en œuvre relativement complexe.

La Demanderesse a découvert que les antigènes glycoprotéiques viraux peuvent être séparés des détergents non ioniques utilisés pour les détacher des virus à l'aide d'une séparation de phases entre une phase aqueuse et une phase constituée par un alcool supérieur insoluble dans l'eau.

De manière surprenante, ce traitement n'altère pas les propriétés des glycoprotéines qui peuvent donc servir à la fabrication d'un vaccin spécifique par exemple contre la grippe.

En conséquence, la présente invention a pour objet un procédé pour isoler les antigènes glycoprotéiques d'un virus par traitement du virus par une solution aqueuse d'un détergent non ionique, séparation des particules virales et élimination du détergent, caractérisé en ce que pour éliminer le détergent on effectue une séparation de phases entre une phase aqueuse et une phase constituée par un alcool ayant au moins 7 atomes de carbone, insoluble dans l'eau et l'on récupère les antigènes glycoprotéiques en phase aqueuse.

Le détergent non ionique peut être tout détergent non ionique utilisé pour détacher les antigènes glycoprotéiques de surface des virus. Le Triton X100E® est classiquement utilisé à cette fin. Il s'agit d'un octylphénoxy polyéthoxyéthanol provenant de la condensation de 9 à 10 molécules d'oxyde d'éthylène sur le p-octylphénol. On peut également utiliser le Triton N101® qui est un nonyl phénoxypolyéthoxyéthanol comprenant 9 à 10 groupes éthoxy.

L'alcool supérieur insoluble dans l'eau peut être tout alcanol ayant au moins 7 atomes de carbone et de préférence 8 atomes de carbone. Il n'y a pas véritablement de limite supérieure si ce n'est pour des questions pratiques, les alcanols en $C_{12}$ et plus étant solides. Le choix de l'alcool supérieur insoluble dans l'eau est guidé par l'étude de son affinité pour le détergent utilisé. Par exemple, si on agite une solution de Triton X100® à 1 % dans un tampon phosphate (contenant NaCL 8,85 g/e, $Na_2HPO_4 12H_2O$ 1, 30 g/e et $NaH_2PO_4$ 0,05 g/e), avec un égal volume de divers alcools supérieurs, on constate, après lavage à l'éther de la phase aqueuse à + 4 °C et évaporation de l'éther, que les quantités résiduelles de Triton X100® dans le tampon mesurées par absorption à 276 nanomètres sont les suivantes :

|  | Triton X100 résiduel |
|---|---|
| Extraction avec alcool décylique primaire | 0,006 % P/V |
| Extraction avec alcool nonylique primaire | 0,005 % P/V |
| Extraction avec alcool octylique primaire | 0,002 % P/V |

L'alcool octylique primaire ou octanol-1 s'avère donc préférable dans le cas du Triton X100®.

Il semble donc que l'affinité de l'alcool pour le détergent est plus grande lorsque les longueurs de chaîne hydrophobe de l'alcanol et du détergent non ionique sont semblables.

Quant à la phase aqueuse, elle peut être constituée par de l'eau ou une solution tampon, par exemple un tampon phosphate contenant du chlorure de sodium à la concentration physiologique.

En pratique, la séparation de phases utilisée dans la présente invention peut être effectuée sur la phase aqueuse obtenue après séparation des particules virales par addition de l'alcool supérieur et élimination de la phase alcoolique après séparation.

Toutefois, dans le cas où l'on utilise du polyéthylène glycol pour séparer les particules virales, les glycoprotéines passent, après addition de l'alcool supérieur, au sein de cet alcool. Il convient alors, après élimination de la phase aqueuse initiale contenant la majorité du polyéthylène glycol, d'ajouter à la phase constituée par l'alcool supérieur une seconde phase aqueuse et d'éliminer la phase alcoolique après séparation des phases.

Pour éliminer les traces d'alcool supérieur de la phase aqueuse, on peut, dans l'un ou l'autre cas, ajouter à la phase aqueuse de l'éther et séparer ultérieurement la phase aqueuse de l'éther.

On décrira maintenant plus en détail le procédé selon la présente invention appliqué à l'isolement de l'hémagglutinine et de la neuraminidase à partir du virus de la grippe.

Le virus de la grippe est cultivé dans l'œuf de poule embryonné. Le liquide allantoïque contenant le virus est récolté. Le virus peut être concentré par diverses méthodes : absorption, élution sur globules rouges de poule formolés et autoclavés, centrifugation, centrifugation en gradients de densité, précipitation par le polyéthylène glycol, etc.

On peut également employer diverses combinaisons de ces méthodes.

L'inactivation du virus par exemple par la β-propiolactone ou le formol peut intervenir à ces stades.

La suspension virale concentrée est additionnée d'un détergent non ionique, par exemple le Triton X100® à une concentration convenable par exemple 2 % volume/volume. Le mélange est incubé à la température ordinaire le temps nécessaire à la solubilisation des glycoprotéines.

Les corpuscules viraux sont ensuite séparés des antigènes glycoprotéiques par un moyen approprié par exemple centrifugation, centrifugation en gradients, « précipitation » ou plutôt exclusion des particules virales par le polyéthylène glycol 6 000, ce dernier procédé étant préféré pour sa commodité.

Après élimination des particules virales, le mélange glycoprotéines-détergent est traité par un volume convenable (en général un égal volume) d'un alcool supérieur insoluble dans l'eau, par exemple l'octanol-1.

Le mélange s'effectue à la température ordinaire (20 °C au moins) par exemple dans une ampoule à décantation. Après mélange pendant un temps suffisant (cinq à dix minutes par exemple) l'émulsion est abandonnée à la température ordinaire. On assiste à la séparation de deux phases : une phase inférieure aqueuse et une phase supérieure formée d'une émulsion résultant de la séparation de l'alcool insoluble. Le détergent non ionique se trouve alors dans l'alcool insoluble dans l'eau. Pour les glycoprotéines, cela dépend de la composition de la phase aqueuse avant son mélange avec l'alcool insoluble.

Cette situation des glycoprotéines dans le système de phases est en particulier influencé par la présence de polymères tels que le polyéthylène glycol 6 000 (PEG 6 000). On peut distinguer en pratique essentiellement deux cas :

Premier cas :
On n'utilise pas le PEG 6 000 pour la séparation des particules virales après action du détergent.
On a recours par exemple à la centrifugation.

Dans ce cas, après élimination des corpuscules viraux, les glycoprotéines solubilisées se trouvent avec le détergent non ionique (par exemple Triton X100® en solution dans un tampon phosphate contenant du chlorure de sodium à la concentration physiologique.

Dans ce cas, le détergent non ionique est retenu dans l'émulsion de la phase supérieure (par exemple l'octanol 1) et les glycoprotéines se retrouvent dans la phase aqueuse inférieure. Après une première décantation, on peut laver la phase alcool avec du tampon qui entraîne les glycoprotéines, le détergent non ionique restant lié à l'alcool supérieur insoluble dans l'eau.

Les phases aqueuses combinées peuvent ensuite être lavées à l'éther pour éliminer les traces d'alcool supérieur dans la phase aqueuse.

Deuxième cas :
Utilisation du polyéthylène glycol.

Après incubation du virus avec le détergent non ionique l'addition d'une quantité convenable de polyéthylène glycol 6 000 (par exemple 8 %) constitue un moyen très commode de séparer les corpuscules viraux des glycoprotéines solubilisées.

Après incubation d'une heure à + 4 °C en présence de PEG 6 000 (par exemple à 8 % P/V), le mélange est centrifugé trente minutes à + 4 °C à 2 000 G.

Si la séparation des corpuscules viraux a lieu à une concentration en PEG 6 000 inférieure à 8 %, on ajoute du PEG 6 000 pour atteindre cette concentration de 8 % avant mélange avec l'alcool supérieur insoluble dans l'eau.

Dans ces conditions, les glycoprotéines quittent la phase aqueuse et migrent dans la phase supérieure alcoolique qui présente comme précédemment l'aspect d'une émulsion.

On élimine alors la phase aqueuse inférieure et on lave la phase supérieure avec une solution tampon (par exemple le tampon phosphate cité plus haut).

On procède par exemple à deux lavages successifs par des volumes de tampon égaux au volume de l'alcool supérieur insoluble dans l'eau et, éventuellement, à un troisième lavage par un volume réduit de tampon.

Comme dans le premier cas, les phases aqueuses réunies peuvent être lavées à l'éther pour éliminer les traces d'alcool supérieur insoluble dans l'eau.

La solution de glycoprotéines ainsi obtenue contient encore du PEG 6 000. L'élimination de ce polymère peut être effectuée par ultrafiltration sous pression sur une membrane de porosité convenable qui permet le passage du PEG 6 000 et retient les glycoprotéines.

On peut aussi réduire considérablement la teneur en PEG 6 000 par addition d'un sel convenable, par exemple le sulfate d'ammonium à raison de 25 grammes de sel ajoutés à 100 ml de solution de glycoprotéines à + 4 °C.

Le PEG 6 000 se sépare alors en une mince couche surnageante que l'on peut séparer par décantation. On élimine le sulfate d'ammonium par dialyse.

On peut combiner ces méthodes : par exemple action d'un sel suivie d'une dialyse sur membrane convenable assurant la dialyse du sel et l'élimination du PEG 6 000 résiduel.

Finalement la solution de glycoprotéines obtenue dans l'un ou l'autre cas peut être stérilisée par filtration sur membrane.

L'extraction des glycoprotéines selon l'invention permet une élimination très efficace du détergent non ionique.

Par exemple, dans le cas de l'emploi du Triton X100® à 2 % pour la solubilisation des glycoprotéines, et de l'octanol-1 (alcool octylique primaire) comme alcool supérieur insoluble dans l'eau, le Triton reste lié à l'octanol et, après lavage à l'éther de la solution de glycoprotéines, on ne retrouve dans cette solution qu'une quantité de Triton X100® inférieure à 0,01 % poids/volume.

Les solutions de glycoprotéines ainsi obtenues peuvent être utilisées pour la préparation de vaccins par simple dilution avec une solution isotonique et notamment un tampon phosphate isotonique.

La présente invention s'applique tout particulièrement à la préparation de vaccin de la grippe (contre les virus de type A ou B). Les vaccins contre la grippe destinés à être administrés à l'homme peuvent être dosés de façon à contenir de 7 à 20 µg d'hémagglutinine.

Les vaccins multivalents sont constitués d'un mélange de glycoprotéines extraites de souches différentes, dont la présence est requise dans le vaccin.

Les glycoprotéines peuvent être administrées en injection sous-cutanée ou intramusculaire telles quelles ou mélangées à un adjuvant immunitaire tel que l'hydroxyde d'aluminium ou le phosphate d'aluminium.

L'invention est illustrée par les exemples suivants :

Exemple 1

Le virus grippal B/Hong Kong/8/73 est cultivé dans la cavité allantoïque d'œufs de poules embryonnés de 11 jours.

Après incubation de 48 heures à 35 °C, les œufs sont mis en chambre froide à + 4 °C pendant une nuit. Le virus contenu dans le liquide allantoïque est concentré par absorption-élution sur globules rouges de poule formolés et autoclavés. Après élution, le virus est mis en suspension dans le tampon suivant :

| | |
|---|---|
| NaCl | 0,85 grammes |
| $PO_4HNa_212H_2O$ | 1,30 grammes |
| $PO_4H_2Na$ | 0,05 grammes |
| Eau distillée QSP | 1 litre |
| le pH est | 7,5 |

Puis, on ajoute 8 % de polyéthylène glycol 6 000 à + 4 °C.

Après une heure, on centrifuge à 2 000 G pendant 30 minutes à + 4 °C.

Le culot est repris dans un volume de tampon égal au 1/10ème de celui de la suspension de départ et contenant 2 % de Triton X100®.

Après incubation de deux heures, à 25 °C, on laisse le mélange toute la nuit à + 4 °C. Le lendemain, on ajoute au mélange 8 % de polyéthylène glycol 6 000.

Après une heure de séjour à + 4 °C, le mélange est centrifugé à 3 000 G pendant 30 minutes à + 4 °C.

Le surnageant est recueilli et, après réchauffement à 22 °C, on mélange avec un égal volume d'octanol-1 dans une ampoule à décanter, on agite pendant dix minutes puis on laisse les phases se séparer à 22 °C pendant une heure. On élimine la phase aqueuse trouble et on la remplace dans l'ampoule par un égal volume de tampon phosphate ayant la composition indiquée ci-dessus et ne contenant pas de polyéthylène glycol. On agite dix minutes et on laisse reposer une heure à 22 °C.

On prélève la phase aqueuse, on la conserve et on renouvelle l'opération. On réunit les deux phases aqueuses, qui sont troubles, on les refroidit à + 4 °C et on les mélange avec un égal volume d'éther froid. On mélange par retournement et agitation ménagée dans une ampoule à décantation, on laisse se séparer les phases pendant la nuit à + 4 °C.

Le lendemain, on sépare la phase aqueuse claire et on en élimine l'éther par évaporation sous vide.

Le polyéthylène glycol est éliminé par ultrafiltration.

La recherche de la quantité de Triton X100® résiduelle dans la solution de glycoprotéines est effectuée en précipitant les glycoprotéines par cinq volumes de méthanol. Après une nuit à la température ordinaire, on centrifuge trente minutes à 2 000 G.

Après élimination du méthanol dans le surnageant par évaporation, on ajoute de l'eau distillée pour amener le résidu au volume initial de l'échantillon et on évalue la quantité de Triton X100® au spectrophotomètre à 276 nanomètres par rapport à un témoin constitué par du tampon ayant subi le même traitement au méthanol, afin d'éliminer l'influence d'éventuelles impuretés du méthanol. Le produit obtenu selon l'invention ne contient que 0,007 % Poids-Volume de Triton® résiduel.

Les glycoprotéines apparaissent pures par électrophorèse en gel de polyacrylamide.

L'examen en microscopie électronique montre des agrégats de glycoprotéines à l'exclusion de tout autre composant.

On a déterminé sur la solution obtenue le titre hémagglutinant, la quantité réelle d'hémagglutinine antigénique par immunodiffusion radiale, ainsi que l'activité de la neuraminidase.

Cette activité enzymatique de la neuraminidase a été recherchée par la méthode de M. AYMARD-HENRY et coll. 1973, Bull. Org. Mond. Sante, Vol. 48, pp. 199-202, par la dilution de la préparation donnant une densité optique de 0,25 à 549 nm.

Titres comparés pour des volumes équivalents de suspension virale d'origine et d'extrait glycoprotéiques :

|  | Suspension virale | Solution de glycoprotéines |
|---|---|---|
| Hémagglutination | 4 020 UI/ml | 3 592 UI/ml |
| Immunodiffusion radiale | 25 μg HA/ml | 20 μg HA/ml |
| Activité de la neuraminidase | 1/200 | 1/110 |

## Exemple 2

On reprend le mode opératoire de l'exemple 1 en utilisant la souche de virus A/Texas/1/77.
On obtient les résultats suivant :
Triton résiduel : 0,007 % P/V

|  | Suspension virale | Solution de glycoprotéines |
|---|---|---|
| Hémagglutination | 4 240 UI/ml | 4 788 UI/ml |
| Immunodiffusion radiale | 60 μg | 27 μg |
| Activité de la neuraminidase | non recherchée | 1/30 |

## Exemple 3

On reprend le mode opératoire de l'exemple 1 en utilisant comme virus la souche recombinante X71 antigéniquement identique à A/Brazil/11/78.
On obtient les résultats suivants :
Triton résiduel : 0,005 % P/V

|  | Suspension virale | Solution de glycoprotéines |
|---|---|---|
| Hémagglutinine | 10 500 UI/ml | 2 500 UI/ml |

## Exemple 4

Le virus grippal A/Victoria/75 est cultivé dans la cavité allantoïque, récolté et concentré par adsorption-élution sur globules rouges de poules formolées et autoclavées comme à l'exemple 1. Il est remis en suspension dans le même tampon et précipité par 8 % de PEG 6 000, le précipité est récolté par centrifugation, remis dans un volume de tampon égal à 1/10ème de celui d'origine contenant 2 % de Triton X100®. Après incubation de deux heures à 25 °C, on laisse le mélange toute la nuit à + 4 °C. Le lendemain, les particules sont éliminées par filtration, ou par centrifugation du surnageant et traitées par un égal volume d'octanol-1, comme à l'exemple 1. Après décantation on recueille la phase aqueuse. On la remplace dans l'ampoule par un égal volume de tampon de même composition, on agite dix minutes et on laisse reposer une heure à 22 °C. On réunit les deux phases aqueuses, on les refroidit à + 4 °C et on les traite par un égal volume d'éther froid, comme dans l'exemple 1. La phase aqueuse claire est enfin recueillie et l'éther est éliminé par évaporation sous vide.

On obtient les résultats suivants :

| | Avant traitement | Après traitement |
|---|---|---|
| Hémagglutinine | 50 µg/ml | 17 µg/ml |
| Neuraminidase | 1/60 | 1/48 |

Triton résiduel : 0,003 %

## Exemple 5

On prépare un vaccin ayant la composition suivante :
Mélange contenant 10 µg d'hémagglutinine obtenue à l'exemple 1.
Tampon phosphate isotonique QSP 1 millilitre.

## Revendications

1. Procédé pour isoler les antigènes glycoprotéiques d'un virus par traitement du virus par une solution aqueuse d'un détergent non ionique, séparation des particules virales et élimination du détergent, caractérisé en ce que pour éliminer le détergent on effectue une séparation de phases entre une phase aqueuse et une phase constituée par un alcool ayant au moins 7 atomes de carbone, insoluble dans l'eau et l'on récupère les antigènes glycoprotéiques en phase aqueuse.

2. Procédé selon la revendication 1, caractérisé en ce que l'alcool insoluble dans l'eau est un alcanol ayant au moins 8 atomes de carbone.

3. Procédé selon la revendication 1 ou la revendication 2, caractérisé en ce que le détergent non ionique est un octylphénoxyéthoxyéthanol provenant de la condensation de 9 à 10 molécules d'oxyde d'éthylène sur le p-octylphénol.

4. Procédé selon la revendication 3, caractérisé en ce que l'alcool insoluble dans l'eau est l'octanol-1.

5. Procédé selon l'une quelconque des revendications précédentes, caractérisé en ce que la phase aqueuse utilisée pour la séparation de phases est celle obtenue après séparation des particules virales en l'absence de polyéthylène glycol.

6. Procédé selon l'une quelconque des revendications 1 à 4, caractérisé en ce que, dans le cas où l'on effectue la séparation des particules virales par le polyéthylène glycol, on ajoute l'alcool insoluble dans l'eau à la phase aqueuse résultante contenant le polyéthylène glycol, les antigènes glycoprotéiques et le détergent non ionique, après séparation des phases on élimine la phase aqueuse, on ajoute une seconde phase aqueuse à la phase constituée par l'alcool insoluble dans l'eau et après séparation des phases on récupère les antigènes glycoprotéiques dans la seconde phase aqueuse.

7. Procédé selon l'une quelconque des revendications 1 à 5, caractérisé en ce que les phases aqueuses séparées de l'alcool insoluble dans l'eau sont lavées à l'éther.

8. Procédé selon l'une quelconque des revendications 1 à 7, caractérisé en ce que la phase aqueuse est constituée par un tampon phosphate.

9. Procédé de préparation d'un vaccin, caractérisé en ce que l'on dilue les antigènes glycoprotéiques obtenus par un procédé selon l'une quelconque des revendications 1 à 7 avec une solution isotonique.

10. Procédé selon la revendication 9, caractérisé en ce que le virus traité est un virus grippal appartenant au type A ou au type B.

11. Procédé selon la revendication 10, caractérisé en ce que les antigènes glycoprotéiques sont dilués de manière à ce que le mélange contienne entre 7 microgrammes et 20 microgrammes d'hémagglutinine de chaque souche de virus.

## Claims

1. Process for the isolation of the glycoproteic antigens of a virus by treatment of said virus with an aqueous solution of a nonionic detergent, separation of the viral particles and removal of the detergent, characterized in that, in order to remove the detergent, a phase separation is effected between an aqueous phase and a phase consisting of a water-insoluble alcohol having at least 7 carbon atoms, and the glycoproteic antigens is recovered in aqueous phase.

2. Process as claimed in claim 1, characterized in that the water-insoluble alcohol is an alkanol having at least 8 carbon atoms.

3. Process as claimed in claim 1 or 2, characterized in that the nonionic detergent is an octyphenoxypolyethoxyethanol derived from the condensation of 9-10 moles ethylene oxide with p-octylphenol.

4. Process as claimed in claim 3, characterized in that said water-insoluble alcohol is 1-octanol.

5. Process as claimed in any one of the preceding claims, characterized in that the aqueous phase used for the phase separation is that obtained after separation of the viral particles in the absence of polyethylene glycol.

6. Process as claimed in any one of the claims 1-4, characterized in that, in the case the viral particle separation is effected with polyethylene glycol, the water-insoluble alcohol is added to the resultant phase containing the polyethylene glycol, the glycoproteic antigens and the nonionic detergent ; the aqueous phase is removed after the phase separation step, a second aqueous phase is added to the phase consisting of the water-insoluble alcohol and, after phase separation, the glycoproteic antigens are recovered in the second aqueous phase.

7. Process as claimed in any one of the claims 1-5, characterized in that the aqueous phases separated from the water-insoluble alcohol are washed with ether.

8. Process as claimed in any one of the claims 1-7, characterized in that the aqueous phase consists of a phosphate buffer.

9. Process for the preparation of a vaccine, characterized in that the glycoproteic antigens obtained by a process as claimed in any one of claims 1-7 are diluted with an isotonic solution.

10. Process as claimed in claim 9, characterized in that the virus treated is a type A or type B influenza virus.

11. Process as claimed in claim 10, characterized in that the glycoproteic antigens are diluted in a manner such that the mixture contains from 7 microgrammes to 20 microgrammes hemagglutinin from each virus strain.


### Ansprüche

1. Verfahren zur Isolierung von Glycoproteid-Antigenen eines Virus durch Behandlung des Virus mit einer wäßrigen Lösung eines nichtionogenen Detergens, Abtrennung der Viruspartikel und Entfernung des Detergens, dadurch gekennzeichnet, daß man zur Entfernung des Detergens eine Trennung von Phasen zwischen einer wäßrigen Phase und einer Phase vornimmt, die aus einem in Wasser unlöslichen Alkohol mit wenigstens 7 C-Atomen besteht, und die Glycoproteid-Antigene in der wäßrigen Phase gewinnt.

2. Verfahren nach Anspruch 1, dadurch gekennzeichnet, daß der in Wasser unlösliche Alkohol ein Alkanol mit wenigstens 8 C-Atomen ist.

3. Verfahren nach Anspruch 1 oder 2, dadurch gekennzeichnet, daß das nichtionogene Detergent ein aus der Kondensation von 9 bis 10 Molekülen Ethylenoxyd mit p-Octylphenol stammendes Octylphenoxyethoxyethanol ist.

4. Verfahren nach Anspruch 3, dadurch gekennzeichnet, daß der in Wasser unlösliche Alkohol Octanol-1 ist.

5. Verfahren nach einem beliebigen der vorstehenden Ansprüche, dadurch gekennzeichnet, daß die für die Trennung der Phasen verwendete wäßrige Phase diejenige ist, die nach der Abtrennung der Viruspartikel in Abwesenheit von Polyethylenglycol erhalten worden ist.

6. Verfahren nach einem beliebigen der Ansprüche 1 bis 4, dadurch gekennzeichnet, daß man in dem Fall, in dem man die Abtrennung der Viruspartikel durch Polyethylenglycol vornimmt, den in Wasser unlöslichen Alkohol der erhaltenen wäßrigen Phase zusetzt, die das Polyethylenglycol, die Glycoproteid-Antigene und das nichtionogene Detergens enthält, nach der Trennung der Phasen die wäßrige Phase entfernt, der durch den in Wasser unlöslichen Alkohol bestehenden Phase eine zweite wäßrige Phase zusetzt und nach Trennung der Phasen die Glycoproteid-Antigene in der zweiten wäßrigen Phase gewinnt.

7. Verfahren nach einem beliebigen der Ansprüche 1 bis 5, dadurch gekennzeichnet, daß die von dem in Wasser unlöslichen Alkohol abgetrennten wäßrigen Phasen mit Ether gewaschen werden.

8. Verfahren nach einem beliebigen der Ansprüche 1 bis 7, dadurch gekennzeichnet, daß die wäßrige Phase aus einem Phosphatpuffer besteht.

9. Verfahren zur Herstellung eines Impfstoffs, dadurch gekennzeichnet, daß man die nach einem Verfahren gemäß einem beliebigen der Ansprüche 1 bis 7 erhaltenen Glycoproteid-Antigene mit einer isotonischen Lösung verdünnt.

10. Verfahren nach Anspruch 9, dadurch gekennzeichnet, daß das behandelte Virus ein zum Typ A oder zum Typ B gehörendes Grippe-Virus ist.

11. Verfahren nach Anspruch 10, dadurch gekennzeichnet, daß die Glycoproteid-Antigene so verdünnt werden, daß das Gemisch zwischen 7 μg und 20 μg Hämagglutinin jedes Virus-Stammes enthält.